# EUROPEAN PATENT APPLICATION

(11) **EP 4 421 176 A1**
(43) Date of publication of application: **28.08.2024**
(21) Application number: 23866703.4
(22) Date of filing: 28.12.2023
(51) Int. Cl.: C12N 15/113, C12N 15/67, C12N 15/85

(54) **NOVEL 5'-UNTRANSLATED REGION ELEMENT AND APPLICATION THEREOF**

(30) Priority: 29.12.2022 CN 202211704246
(71) Applicant: Themedium Therapeutics Co., Ltd, Suzhou, Jiangsu 215011 (CN)
(72) Inventor: KANG, Tao, Suzhou, Jiangsu 215011 (CN); ZHANG, Wenjing, Suzhou, Jiangsu 215011 (CN); ZHANG, Jingming, Suzhou, Jiangsu 215011 (CN)
(74) Representative: LLR
(86) International application number: PCT/CN2023/142894
(87) International publication number: WO 2024/140950

(57) **Abstract**

The present application provides a novel 5'-untranslated region element, wherein the 5'-untranslated region element comprises the full-length sequence of the 5'-untranslated region of a naturally highly expressed gene; or a fragment sequence of the 5'-untranslated region of a naturally highly expressed gene, wherein the fragment sequence of the 5'-untranslated region does not comprise a TOP motif site (e.g., CTTTT) or a uORF sequence (AUG upstream of an open reading frame); or a fragment sequence of the 5'-untranslated region of a naturally highly expressed gene, a non-structural sequence, a transcription-enhancing sequence, and a Kozac sequence, wherein the fragment sequence of the 5'-untranslated region does not comprise a TOP motif site or a uORF sequence. The 5'-untranslated region element of the present application makes nucleic acid molecules comprising same have extremely high protein expression performance and expression efficiency. Therefore, the element can be used for constructing artificial nucleic acid molecules with high expression performance and has excellent application prospects in the development of nucleic acid therapeutic drugs.

## Description

### CROSS-REFERENCE

The present application claims priority to Chinese Patent Application No. 2022117042468 filed on Dec. 29, 2022 and titled "NOVEL 5'-UNTRANSLATED REGION ELEMENT AND USE THEREOF", which is incorporated herein by reference in its entirety.

### TECHNICAL FIELD

The present application relates generally to the field of molecular biology. More specifically, it relates to a novel artificial 5'-untranslated region element (5'-UTR) and an artificial nucleic acid molecule comprising same, and use of the novel artificial 5'-untranslated region element (5'-UTR) and the artificial nucleic acid molecule comprising same in protein expression.

### BACKGROUND

Therapeutic or prophylactic nucleic acids have the potential to revolutionize vaccination, gene therapy, protein alternative therapy, and other therapies for genetic diseases. Since the first clinical study on therapeutic nucleic acids in 2000, significant advances have been made in the research on the design of nucleic acid molecules and the method of delivering them. However, nucleic acid drugs (including therapeutic and prophylactic drugs) are still confronted by several challenges, including drug delivery, druggability, etc.

In the process of combating COVID-19, mRNAs have been clinically and commercially validated as novel vaccines and have also shown great potential in protein supplementation therapy, gene editing, cell therapy, etc. mRNA vaccines or drugs are introduced in the form of mRNA sequences into cells and function by producing proteins of interest using the protein translation machinery of the cells. The 5' cap, UTR, polyAtail, CDS, non-natural nucleotide modifications, etc. of an mRNA are all closely related to mRNA translation efficiency and mRNA stability. Two important functions of 5' UTRs are to stabilize mRNA molecules and to assist ribosomal subunits in locating start codons. Therefore, they are crucial for regulating mRNA translation efficiency and mRNA stability. Main mechanisms for fulfilling the functions of 5' UTRs described above, for example, (1) the upstream open reading frames (uORFs) of 5' UTRs being capable of regulating the expression levels of specific genes and the types of expression product variants; (2) the secondary structures of 5' UTRs hindering the 5'-3' degradation activity of nucleases; (3) the differences in the affinity activity of the 5' UTR ACAC non-structural short sequence for ribosomes; and (4) 5' UTR sequences ranging from several nt to several thousand nt in length, provide another level of regulatory mechanism diversity for mRNA translation efficiency and stability.

The UTRs of natural genes have diversity in their sequences and higher-order structures, and the 5' UTRs of naturally highly expressed genes naturally have more advantages in terms of sequence and structure, which offer more possibilities for developing novel UTR sequences that can be used for vaccines or drugs.

### SUMMARY

The present application firstly provides a novel artificial 5'-untranslated region element, wherein the 5'-untranslated region element is formed by operably linking the following motifs: a transcription-enhancing sequence (e.g., AATAA), a Kozac sequence (GCCACC), and the full-length sequence of the 5'-untranslated region of a naturally highly expressed gene; or a transcription-enhancing sequence, a Kozac sequence, and a fragment sequence of the 5'-untranslated region of a naturally highly expressed gene, wherein the fragment sequence of the 5'-untranslated region of the naturally highly expressed gene does not comprise a TOP motif site (e.g., CTTTT or another TOP motif site sequence disclosed in the art) or a uORF sequence (the "ATG" upstream of an open reading frame); or a transcription-enhancing sequence, a Kozac sequence, a non-structural sequence (e.g., a nucleic acid chain sequence that consists of only the nucleotides A, T, and C and is theoretically incapable of forming a secondary structure), and a fragment sequence of the 5'-untranslated region of a naturally highly expressed gene, wherein the fragment sequence of the 5'-untranslated region of the naturally highly expressed gene does not comprise a TOP motif site (e.g., CTTTT or another TOP motif site sequence disclosed in the art) or a uORF sequence (the "AUG" upstream of an open reading frame). The order in which the motifs described above are linked in the direction from 5' to 3' may be:
(1) the transcription-enhancing sequence, the full-length sequence of the 5'-untranslated region of the naturally highly expressed gene, and the Kozac sequence; or
(2) the transcription-enhancing sequence, the fragment sequence of the 5'-untranslated region of the naturally highly expressed gene free of the TOP motif site (e.g., CTTTT) and the uORF sequence (the "AUG" upstream of the open reading frame), and the Kozac sequence; or
(3) the transcription-enhancing sequence, the fragment sequence of the 5'-untranslated region of the naturally highly expressed gene free of the TOP motif site (e.g., CTTTT) and the uORF sequence (the "AUG" upstream of the open reading frame), the non-structural sequence, and the Kozac sequence.

The naturally highly expressed gene described above may be derived from any species; it may be a gene derived from a mammal such as a human, or a gene derived from a microbe such as a virus or a bacterium. For example, it may be the human ribosomal protein S25 gene, the human actin β gene, the human hemoglobin subunit α gene, the human hemoglobin subunit β gene, the human complement factor 3 gene, the human cytochrome B-245 α gene, the human TM4SF1 gene, the human TXNIP gene, the human RPS11 gene, or the like, or a mutant thereof. In a preferred embodiment, the candidate 5' UTR sequence selected by the present application is mainly derived from a highly expressed gene of human cells, such as the ribosomal protein S25 gene, the ribosomal protein S11 gene, the actin β gene, the hemoglobin subunit α gene, the hemoglobin subunit β gene, the thioredoxin interacting protein (TXNIP) gene, the complement factor 3 gene, the cytochrome B-245 α gene, the human transmembrane-4 L-six family member-1 (TM4SF1) gene, or the like. The mutant is cut from the 5' end, the 3' end, or any segment between the 5' end and the 3' end of the 5' UTR of the natural gene; the mutant is 50 nt to 200 nt (nucleotides) in length; the TOP motif therein is mutated into CTTT or CTT or CT or C; the uORF sequence of ATG therein is mutated into ATT or the three nucleotides ATG are completely deleted; no other sequence exists at the 5' end and/or the 3' end of the mutant or a non-structural nucleic acid sequence is linked to the 5' end and/or the 3' end of the mutant.

The fragment sequence of the 5'-untranslated region of the naturally highly expressed gene is cut from the 5' end, the 3' end, or any segment between the 5' end and the 3' end of the 5' UTR of the natural gene; the fragment is 50 nt to 200 nt (nucleotides) in length; in the fragment, the TOP motif site is mutated into CTTT or CTT or CT or C; in the fragment, a uORF sequence such as ATG is mutated into ATT or the three nucleotides ATG are completely deleted; no other sequence exists at the 5' end and/or the 3' end of the fragment; a non-structural nucleic acid sequence is operably linked to the 5' end and/or the 3' end of the fragment, and the linking may be direct or by a linker. In the novel artificial 5'-untranslated region element provided by the present application, the transcription-enhancing sequence may be selected from AATAA, ATAAT, or AATAAA; the Kozac sequence is selected from GCCACC; the non-structural sequence refers to a nucleic acid chain sequence that consists of only the nucleotides A, T, and C and is theoretically incapable of forming a secondary structure, or a nucleotide sequence that is 30 nt to 50 nt in length, takes "AC" as a backbone and a main component, and does not comprise "G", e.g., a sequence that does not comprise G and comprises 80% or more AC and 20% or less T; preferably, the non-structural sequence is selected from CACACCCACTACACCTACACATATCTACTCACCTACACACTAATA (SEQ ID No. 21) or CATACCCAAATTTATATCTACATCTAAACCCAATTATTACCC (SEQ ID No. 22) or ACCACACACTTATCTACACAACTCAATCC (SEQ ID No. 23) or TCCACCCAACCCACTACTAATACCCACAACCCAACACCC (SEQ ID No. 24) or AACCCACACACTCACCTACTCATCCA (SEQ ID No. 25); more preferably, the non-structural sequence is selected from CACACCCACTACACCTACACATATCTACTCACCTACACACTAATA (SEQ ID No. 21).

In another aspect, the present application further provides an artificial nucleic acid molecule, and the artificial nucleic acid molecule comprises:
at least one of the aforementioned 5'-untranslated region elements;
at least one open reading frame; and
at least one 3'-untranslated region element.

The artificial nucleic acid molecule described above may comprise natural nucleotides, or nucleotide mimics or functional analogs, or chemically modified forms of nucleotides.

Functional nucleotide analogs include, but are not limited to, one of or a combination of more than one of a locked nucleic acid (LNA), a peptide nucleic acid (PNA), and a morpholine ring oligonucleotide nucleic acid mimic or functional analog.

The chemical modification of nucleotides may be located on a backbone bond of the nucleic acid molecule. The backbone bond may be modified by replacement of one or more oxygen atoms. The modification to the backbone bond may include replacing at least one phosphodiester bond with a phosphorothioate bond.

The chemical modification of nucleotides may be located on a nucleoside. The modification on the nucleoside may be located on the sugar and base of the nucleoside. The sugar on the nucleoside may be selected from one or more of 2'-fluororibose, ribose, 2'-deoxyribose, arabinose, and hexose.

The chemically modified forms of nucleotides may be selected from one or more of 5-methylcytosine, pseudouridine, 1-methylpseudouridine, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonylcarbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine. These modifications may be random or at specific sites.

Further, the artificial nucleic acid molecule described above may comprise a nucleic acid sequence encoding a signal peptide; still further, the nucleic acid molecule described above may comprise a poly(A) sequence or a poly(A) signal sequence.

All nucleic acid elements may be linked directly or by linkers.

In a third aspect, the present application further provides a vector comprising the artificial nucleic acid molecule described above.

In a fourth aspect, the present application further provides a host cell comprising the vector described above.

In a fifth aspect, the present application further provides an RNA obtained by transcription using the artificial nucleic acid molecule or vector described above, wherein the RNA may be a self-amplification RNA, a long non-coding RNA (LncRNA), a circular RNA (circRNA), or an mRNA, and the RNA is preferably an mRNA.

In a sixth aspect, the present application further provides a protein or polypeptide encoded by the artificial nucleic acid molecule or vector or RNA described above, wherein the protein or polypeptide may be a prophylactic protein or polypeptide or a therapeutic protein or polypeptide. In a preferred instance, the artificial nucleic acid molecule or vector or RNA described above can encode at least one antigen or a fragment thereof or an epitope thereof, and the antigen is selected from a pathogenic antigen such as a tumor-associated antigen or a pathogenic microbial antigen. In another preferred instance, the artificial nucleic acid molecule or vector or RNA described above can encode at least one therapeutic protein or polypeptide, including protein molecules in a broad sense such as cytokines, chemokines, interleukins, interferons, growth factors, and blood coagulation factors, anticoagulants, blood factors, bone morphogenetic proteins, immunoglobulins, enzymes, etc.

The present application further provides use of the artificial nucleic acid molecule or vector or host cell or RNA described above or the encoded protein or polypeptide thereof in the preparation of a medicament.

The present application further provides a medicament, wherein the medicament comprises the artificial nucleic acid molecule or vector or host cell or RNA described above or the encoded protein or polypeptide thereof and a pharmaceutically acceptable carrier or auxiliary material. Pharmaceutical auxiliary materials refer to excipients and additives used in drug production and prescription dispensing and are substances other than the active ingredient, which have been rationally evaluated in safety and are contained in a pharmaceutical formulation. In addition to shaping, serving as vehicles, and enhancing stability, pharmaceutical auxiliary materials also have such important functions as solubilization, aiding dissolution, sustaining or controlling release, etc. They are important ingredients which possibly affect the quality, safety and effectiveness of drugs. According to the effect and the purpose, pharmaceutical auxiliary materials may be classified into solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adhesives, integrating agents, permeation promotors, pH regulators, buffers, plasticizers, surfactants, foaming agents, defoaming agents, thickeners, inclusion agents, humectants, absorbents, diluents, flocculants, deflocculants, filter aids, release retardants, etc.

The present application further provides use of the artificial nucleic acid molecule, the vector, the host cell, the protein, or the polypeptide in the preparation of the medicament described above. Beneficial Effects of The Present Application:
The present application provides a novel 5'-untranslated region element; nucleic acid molecules comprising the novel UTR of the present application have at least 25%, at least 50%, at least 75%, at least 100%, or at least 150% higher protein expression performance and at least 50%, at least 100%, at least 150%, at least 200%, at least 250%, at least 300%, at least 350%, at least 400%, at least 500%, at least 600%, at least 700%, or at least 800% higher expression efficiency than those comprising UTRs disclosed in the prior art.

### BRIEF DESCRIPTION OF THE DRAWINGS

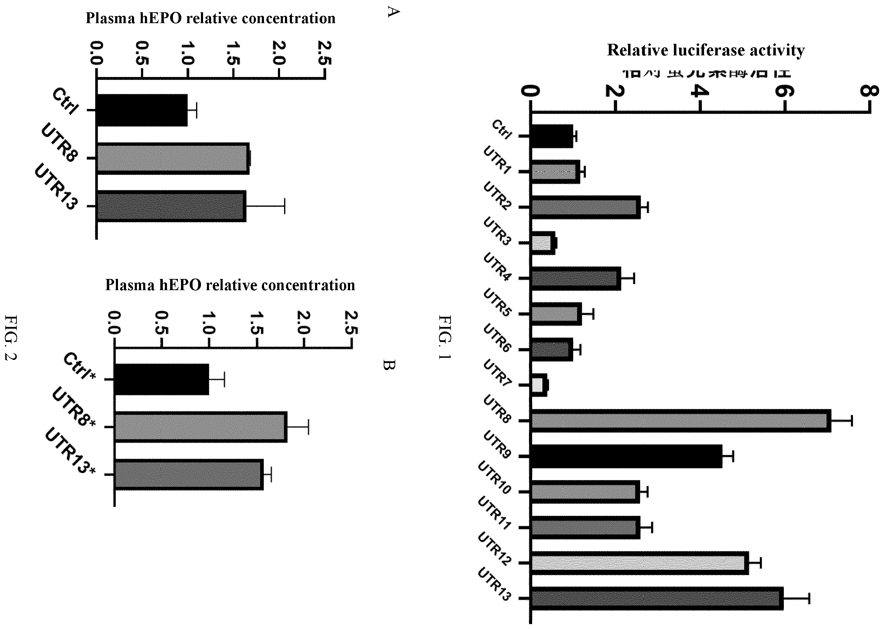

FIG. 1 shows the relative luciferase activity assay results for candidate UTRs; compared to the control UTR (Ctrl in FIG. 1, BNT162b2 5' UTR) and UTR1, UTR2 and UTR4 showed more than 2 times higher luciferase expression levels than the control group, and UTR8 and UTR9 showed more than 4 times and even nearly 8 times higher luciferase expression levels than the control group; UTR10, UTR11, and UTR12 showed 2-4 times higher luciferase expression levels than the control UTR; UTR13 showed 4-6 times higher luciferase expression levels than the control UTR. Panel A in FIG. 2 shows assays for the levels of plasma hEPO expression driven by candidate UTRs. LNP-encapsulated test UTR-mRNAs were injected into the tail veins of mice. After 6 hours, plasma was collected and assayed for plasma hEPO concentration by ELISA. UTR8 and UTR13 showed nearly 2 times higher protein expression relative concentrations than the control UTR (Ctrl: BNT162b2 5' UTR and the 3' UTR of the HBA1 gene). Panel B shows assays for the levels of plasma hEPO expression driven by candidate 5' UTRs combined with BNT162b2 3' UTR. Ctrl* represents BNT162b2 5' UTR + 3' UTR. When combined with BNT162b2 3' UTR, UTR8 and UTR13 showed higher protein expression relative concentrations.

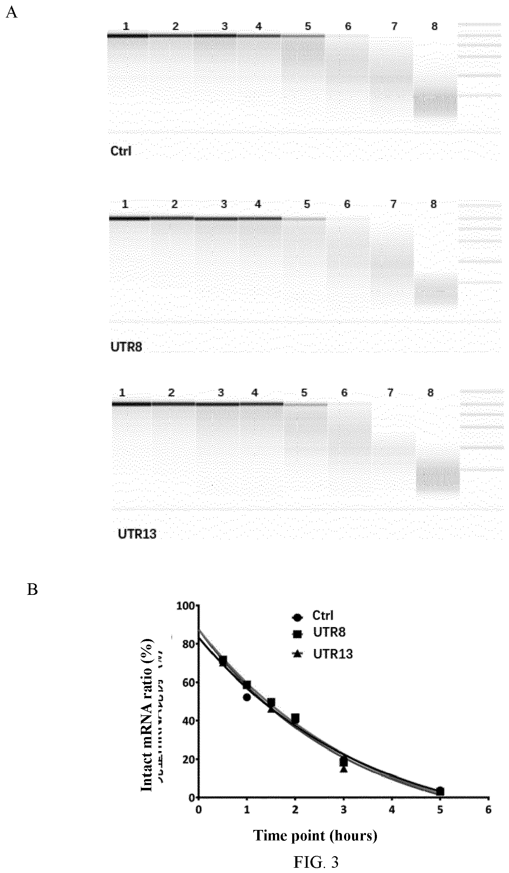

FIG. 3 shows candidate UTR-mRNA solution stability tests. Panel A shows the results of mRNA integrity tests using capillary electrophoresis (Agilent 5200); panel B shows a quantitative curve chart, and the results show that the control and candidate UTR mRNAs have similar half-lives in solution; wherein Ctrl represents the control UTR; the numbers 1-8 in panel A represent the time points 0.5 h, 1 h, 1.5 h, 2 h, 3 h, 5 h, 9 h, and 24 h, respectively.

### DETAILED DESCRIPTION

### A. Definitions

"Prevention" or "preventing" includes: (1) inhibiting the onset of a disease in a subject or patient who may be at risk of and/or susceptible to the disease, but who has not yet experienced or exhibited any or all of the conditions or symptoms of the disease; and/or (2) slowing the onset of the conditions or symptoms of a disease in a subject or patient who may be at risk of and/or susceptible to the disease, but who has not experienced or exhibited any or all of the conditions or symptoms of the disease.

"Treatment" or "treating" includes: (1) inhibiting a disease (e.g., arresting the further development of the conditions and/or symptoms) in a subject or patient experiencing or exhibiting the conditions or symptoms of the disease, (2) ameliorating a disease (e.g., reversing the conditions and/or symptoms) in a subject or patient experiencing or exhibiting the conditions or symptoms of the disease, and/or (3) effecting any measurable mitigation of a disease in a subject or patient experiencing or exhibiting the conditions or symptoms of the disease.

"Protein", "polypeptide" or "peptide" refers to a polymer of amino acid residues, including a wide range of protein molecules such as cytokines, chemokines, interleukins, interferons, growth factors, blood coagulation factors, anticoagulants, blood factors, bone morphogenic proteins, immunoglobulins, and enzymes. Some non-limiting examples of therapeutic proteins include the following therapeutic proteins or fragments, variants, or derivatives thereof: therapeutic proteins for treating metabolic or endocrine disorders, including acid sphingomyelinase, adipotide, agalsidase-β, alglucosidase, α-galactosidase A, α-glucosidase, α-L-iduronidase, α-N-acetylglucosaminidase, amphiregulin, angiopoietins (Ang1, Ang2, Ang3, Ang4, ANGPTL2, ANGPTL3, ANGPTL4, ANGPTL5, ANGPTL6, ANGPTL7), β cellulin, β-glucuronidase, bone morphogenetic proteins BMPs (BMP1, BMP2, BMP3, BMP4, BMP5, BMP6, BMP7, BMP8a, BMP8b, BMP10, BMP15), CLN6 protein, epidermal growth factor (EGF), epigen, epiregulin, fibroblast growth factors (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23), galsulphase, ghrelin, glucocerebrosidase, GM-CSF, heparin-binding EGF-like growth factor (HB-EGF), hepatocyte growth factor HGF, hepcidin, human albumin, increased loss of albumin, idursulphase (iduronate-2-sulphatase), integrins αVβ3, αVβ5 and α5β1, iuduronate sulfatase, laronidase, N-acetylgalactosamine-4-sulfatase (rhASB; galsulfase, aryl sulfatase A (ARSA), aryl sulfatase B (ARSB)), N-acetylglucosamine-6-sulfatase, nerve growth factor (NGF, brain-derived neurotrophic factor (BDNF), neurotrophin-3 (NT-3), and neurotrophin 4/5 (NT-4/5), neuregulins (NRG1, NRG2, NRG3, NRG4), neuropilins (NRP-1, NRP-2), obestatin, platelet derived growth factors (PDGFs (PDFF-A, PDGF-B, PDGF-C, PDGF-D), TGFβ receptors (endoglin, TGF-β1 receptor, TGF-β2 receptor, TGF-β3 receptor), thrombopoietin (THPO) (megakaryocyte growth and development factor (MGDF)), transforming growth factors (TGFs (TGF-a, TGF-β (TGFβ1, TGFβ2, and TGFβ3))), VEGF (VEGF-A, VEGF-B, VEGF-C, VEGF-D, VEGF-E, VEGF-F, and PIGF), nesiritide, trypsin, adrenocorticotrophic hormone (ACTH), atrial-natriuretic peptide (ANP), cholecystokinin, gastrin, leptin, oxytocin, somatostatin, vasopressin (antidiuretic hormone), calcitonin, exenatide, growth hormone (GH), somatotropin, insulin, insulin-like growth factor 1 IGF-1, mecasermin rinfabate, IGF-1 analog, mecasermin, IGF-1 analog, pegvisomant, pramlintide, teriparatide (human parathyroid hormone residues 1-34), becaplermin, diboternnin-α (bone morphogenetic protein 2), histrelin acetate (gonadotropin releasing hormone; GnRH), octreotide, and palifermin (keratinocyte growth factor; KGF); therapeutic proteins for treating blood disorders, diseases of the circulatory system, diseases of the respiratory system, cancer or tumour diseases, infectious diseases, or immunedeficiencies, including alteplase (tissue plasminogen activator; tPA), anistreplase, antithrombin III (AT-III), bivalirudin, darbepoetin-α, drotrecogin α (activated protein C, erythropoietin, epoetin-α, erythropoetin, erthropoyetin, factor IX, factor VIIa, factor VIII, lepirudin, protein C concentrate, reteplase (deletion mutein of tPA), streptokinase, tenecteplase, urokinase, angiostatin, anti-CD22 immunotoxin, denileukin diftitox, Immunocyanin, MPS (metallopanstimulin), aflibercept, endostatin, collagenase, human deoxy-ribonuclease I, domase, hyaluronidase, papain, L-asparaginase, PEG-asparaginase, rasburicase, human chorionic gonadotropin (HCG), human follicle-stimulating hormone (FSH), lutropin-α, prolactin, α-1-proteinase inhibitor, lactase, pancreatic enzymes (lipase, amylase, protease), adenosine deaminase (pegademase bovine, PEG-ADA), abatacept, alefacept, anakinra, etanercept, interleukin-1 (IL-1) receptor antagonist, anakinra, thymulin, TNF-α antagonist, enfuvirtide, and thymosin α1; therapeutic proteins selected from adjuvant or immunostimulating proteins, including: human adjuvant proteins, particularly pattern recognition receptors TLR1, TLR2, TLR3, TLR4, TLR5, TLR6, TLR7, TLR8, TLR9, TLR10, TLR11; NOD1, NOD2, NOD3, NOD4, NOD5, NALP1, NALP2, NALP3, NALP4, NALP5, NALP6, NALP6, NALP7, NALP7, NALP8, NALP9, NALP10, NALP11, NALP12, NALP13, NALP14, 1IPAF, NAIP, CIITA, RIG-I, MDA5, and LGP2, signal transducers of TLR signaling, including adaptor proteins, including, e.g., Trif and Cardif; components of small-GTPase signaling (RhoA, Ras, Rac1, Cdc42, Rab, etc.), components of PIP signaling (PI3K, Srckinases, etc.), components of MyD88-dependent signaling (MyD88, IRAK1, IRAK2, IRAK4, TIRAP, TRAF6, etc.), components of MyD88-independent signaling (TICAM1, TICAM2, TRAF6, TBK1, IRF3, TAK1, IRAK1, etc.); activated kinases, including, e.g., Akt, MEKK1, MKK1, MKK3, MKK4, MKK6, MKK7, ERK1, ERK2, GSK3, PKC kinases, PKD kinases, GSK3 kinases, JNK, p38MAPK, TAK1, IKK, and TAK1; activated transcription factors, including, e.g., NF-κB, c-Fos, c-Jun, c-Myc, CREB, AP-1, Elk-1, ATF2, IRF-3, IRF-7, heat shock proteins, such as HSP10, HSP60, HSP65, HSP70, HSP75, and HSP90, gp96, fibrinogen, TypIII repeat extra domain A of fibronectin; or components of the complement system, including C1q, MBL, C1r, C1s, C2b, Bb, D, MASP-1, MASP-2, C4b, C3b, C5a, C3a, C4a, C5b, C6, C7, C8, C9, CR1, CR2, CR3, CR4, C1qR, C1INH, C4bp, MCP, DAF, H, I, P, and CD59, or induced target genes, including, e.g., β-defensin, cell surface proteins; or human adjuvant proteins, including trif, flt-3 ligand, Gp96, or fibronectin, cytokines that induce or enhance an innate immune response, including IL-1α, IL1β, IL-2, IL-6, IL-7, IL-8, IL-9, IL-12, IL-13, IL-15, IL-16, IL-17, IL-18, IL-21, IL-23, TNFα, IFNα, IPNβ, IPNγ, GM-CSF, G-CSF, M-CSF; chemokines, including IL-8, IP-10, MCP-1, MIP-1α, RANTES, eotaxin, CCL21; cytokines released by macrophages, including IL-1, IL-6, IL-8, IL-12, and TNF-α; as well as IL-1R1 and IL-1α, bacterial (adjuvant) proteins, particularly bacterial heat shock proteins or chaperones, including Hsp60, Hsp70, Hsp90, Hsp100; OmpA (outer membrane protein) from gram-negative bacteria; bacterial porins, including OmpF; bacterial toxins, including pertussis toxin (PT) from Bordetella pertussis, pertussis adenylate cyclase toxin CyaA and CyaC from Bordetella pertussis, PT-9K/129G mutant from pertussis toxin, pertussis adenylate cyclase toxin CyaA and CyaC from Bordetella pertussis, tetanus toxin, cholera toxin (CT), cholera toxin B-subunit, CTK63 mutant from cholera toxin, CTE112K mutant from CT, Escherichia coli heat-labile enterotoxin (LT), B subunit from heat-labile enterotoxin (LTB), Escherichia coli heat-labile enterotoxin mutants with reduced toxicity, including LTK63, LTR72; phenol-soluble modulin; neutrophil-activating protein (HP-NAP) from Helicobacter pylori; surfactant protein D; outer surface protein A lipoprotein from Borrelia burgdorferi, Ag38 (38 kDa antigen) from Mycobacterium tuberculosis; proteins from bacterial fimbriae; enterotoxin CT of Vibrio cholerae, pilin from the pili of gram-negative bacteria, and surfactant protein A and bacterial flagellins, protozoan (adjuvant) proteins, particularly Tc52 from Trypanosoma cruzi, PFTG from Trypanosoma gondii, protozoan heat shock proteins, LeIF from Leishmania spp., profilin-like protein from Toxoplasma gondii, viral (adjuvant) proteins, particularly respiratory syncytial virus fusion glycoprotein (F-protein), envelope protein from MMT virus, mouse leukemia virus protein, hemagglutinin protein of wild-type measles virus, fungal (adjuvant) proteins, particularly fungal immunomodulatory protein (FIP; LZ-8); and keyhole limpet hemocyanin (KLH), OspA; therapeutic proteins for hormone replacement therapy, particularly oestrogens, progesterone or progestins, and testosterone; therapeutic proteins for reprogramming somatic cells into pluri- or omnipotent stem cells, particularly Oct-3/4, Sox gene family (Sox1, Sox2, Sox3, and Sox15), Klf family (Klf1, Klf2, Klf4, and Klf5), Myc family (c-myc, L-myc, and N-myc), Nanog, and LIN28; therapeutic antibodies selected from antibodies for treating cancer or tumor diseases, particularly 131T-tositumomab, 3F8, 8H9, abagovomab, adecatumumab, afutuzumab, alacizumab pegol, alemtuzumab, amatuximab, AME-133v, AMG102, anatumomab mafenatox, apolizumab, bavituximab, bectumomab, belinnumab, bevacizumab, bivatuzumab-DM1, blinatumomab, brentuximab vedotin, cantuzumab, cantuzumab mertansine, cantuzumab ravtansine, capromab pendetide, carlumab, catumaxomab, cetuximab, citatuzumab bogatox, cixutumumab, clivatuzumab tetraxetan, CNTO328, CNTO 95, conatumumab, dacetuzumab, dalotuzumab, denosumab, detumomab, drozitumab, ecromeximab, edrecolomab, elotuzumab, elsilimomab, enavatuzumab, ensituximab, epratuzumab, ertumaxomab, ertumaxomab, etaracizumab, farletuzumab, FBTA05, ficlatuzumab, figitumumab, flanvotumab, galiximab, galiximab, ganitumab, GC1008, gemtuzumab, gemtuzumab ozogamicin, girentuximab, glembatumumab vedotin, GS6624, HuC242-DM4, HuHMFG1, HuN901-DM1, ibritumomab, icrucumab, ID09C3, indatuximab ravtansine, inotuzumab ozogamicin, intetumumab, ipilimumab, iratumumab, labetuzumab, lexatumumab, lintuzumab, lorvotuzumab mertansine, lucatumumab, lumiliximab, mapatumumab, matuzumab, MDX-060, MEDI 522, mitumomab, mogamulizumab, MORab-003, MORab-009, moxetumomab pasudotox, MT103, nacolomab tafenatox, naptumomab estafenatox, narnatumab, necitumumab, nimotuzumab, nimotuzumab, olaratumab, onartuzumab, oportuzumab monatox, oregovomab, oregovomab, PAM4, panitumumab, patritumab, pemtumomab, pertuzumab, pritumumab, racotumomab, radretumab, ramucirumab, rilotumumab, rituximab, robatumumab, samalizumab, SGN-30, SGN-40, sibrotuzumab, siltuximab, tabalumab, tacatuzumab tetraxetan, taplitumomab paptox, tenatumomab, teprotumumab, TGN1412, ticilimumab (=tremelimumab), tigatuzumab, TNX-650, tositumomab, trastuzumab, TRBS07, tremelimumab, TRU-016, TRU-016, tucotuzumab celmoleukin, ublituximab, urelumab, veltuzumab, veltuzumab (IMMU-106), volociximab, votumumab, WX-G250, zalutumumab, and natalizumab; antibodies for treating immune disorders, particularly efalizumab, epratuzumab, etrolizumab, fontolizumab, ixekizumab, mepolizumab, milatuzumab, pooled immunoglobulins, priliximab, rituximab, rontalizumab, ruplizumab, sarilumab, vedolizumab, visilizumab, reslizumab, adalimumab, aselizumab, atinumab, atlizumab, bertilimumab, besilesomab, BMS-945429, briakinumab, brodalumab, canakinumab, canakinumab, certolizumab pegol, erlizumab, fezakinumab, golimumab, gomiliximab, infliximab, mavrilimumab, natalizumab, ocrelizumab, odulimomab, ofatumumab, ozoralizumab, pexelizumab, rovelizumab, SBI-087, SBI-087, secukinumab, sirukumab, talizumab, tocilizumab, toralizumab, TRU-015, TRU-016, ustekinumab, ustekinumab, vepalimomab, zolimomab aritox, sifalimumab, lumiliximab, and Rho(D) immunoglobulin; antibodies for treating infectious diseases, particularly afelimomab, CR6261, edobacomab, efungumab, exbivirumab, felvizumab, foravirumab, ibalizumab, libivirumab, motavizumab, nebacumab, tuvirumab, urtoxazumab, bavituximab, pagibaximab, palivizumab, panobacumab, PRO140, rafivirumab, raxibacumab, regavirumab, sevirumab, suvizumab, and tefibazumab; antibodies for treating blood disorders, particularly abciximab, atorolimumab, eculizumab, mepolizumab, and milatuzumab; antibodies for immunoregulation, particularly antithymocyte globulin, basilixinnab, cedelizumab, daclizumab, gavilimomab, inolinnomab, muromonab-CD3, muromonab-CD3, odulimomab, and siplizumab; antibodies for treating diabetes, particularly gevokizumab, otelixizumab, and teplizumab; antibodies for treating alzheimer's disease, particularly bapineuzumab, crenezumab, gantenerumab, ponezumab, R1450, and solanezumab; antibodies for treating asthma, particularly benralizumab, enokizumab, keliximab, lebrikizumab, omalizumab, oxelumab, pascolizumab, and tralokinumab; antibodies for treating a variety of disorders, particularly blosozumab, CaroRx, fresolimumab, fulranumab, romosozumab, stamulumab, tanezumab, and ranibizumab; erythropoietin (EPO), granulocyte colony-stimulating factor (G-CSF), α-galactosidase A, α-L-iduronidase, thyrotropin α, N-acetylgalactosamine-4-sulfatase (rhASB), domase alfa, tissue plasminogen activator (TPA) activase, glucocerebrosidase, interferon (IF) β-1α, interferon β-1b, interferon γ, interferon α, TNF-α, IL-1 to IL-36, human growth hormone (rHGH), human insulin (BHI), human chorionic gonadotropin α, darbepoetin α, follicle-stimulating hormone (FSH) and factor VIII, antibodies and antibody derivatives such as bispecific antibodies, multispecific antibodies, ADCs, etc. for treating a variety of disorders. A peptide is a compound formed by linking α-amino acids by peptide bonds, and it is also an intermediate product of protein hydrolysis. Generally, the number of amino acids contained in a peptide is two to nine, and peptides are variously referred to depending on the number of amino acids in the peptides: dipeptide, tripeptide, tetrapeptide, pentapeptide, etc. Peptides consisting of three or more amino acid molecules are known as polypeptides, which have a molecular weight of less than 10,000 Da, being capable of passing through a semipermeable membrane without being precipitated by trichloroacetic acid and ammonium sulfate. In some literature, peptides consisting of 2-10 amino acids are referred to as oligopeptides (small-molecule peptides); peptides consisting of 10-50 amino acids are referred to as polypeptides; peptides consisting of 50 or more amino acids are referred to as proteins. In other words, proteins are sometimes referred to as polypeptides.

Antigens (abbreviated as Ag) refer to substances capable of causing antibody production and are any substances capable of inducing an immune response. Foreign molecules may be recognized by immunoglobulin on B cells, or treated by antigen-presenting cells, combined with a major histocompatibility complex to obtain a complex to reactivate T cells and trigger a continuous immune response.

An antigenic epitope, also known as an antigenic determinant, may be a special chemical group which consists of a continuous sequence (a protein primary structure) or a discontinuous protein three-dimensional structure and determines antigenicity. Most of antigenic epitopes are present on the surface of antigenic materials, and some of the antigenic epitopes are present inside the antigenic materials, and are exposed after treatment with enzymes or other means. One natural antigenic substance may have a variety of and a plurality of determinants. The larger the molecule of an antigen, the greater the number of the epitopes.

Tumor-associated antigens (TAA) refer to antigen molecules present on tumor cells or normal cells, including: embryonic proteins, glycoprotein antigens, squamous cell antigens, and the like, and are commonly used for clinical diagnosis of tumors. The tumor-associated antigens are not specific to the tumor cells, may be synthesized in minute amounts on the normal cells, are highly expressed when the tumor cells proliferate, and are therefore referred to as "associated antigens". Tumors derived from the same tissue type have the same tumor-associated antigens in different individuals.

Pathogenic microbes, also known as pathogens, refer to microbes that can invade human bodies and cause infections and even infectious diseases. Bacteria and viruses are the most harmful of antigens. Pathogenic microbes refer to prion bodies, fungi, bacteria, spirochetes, mycoplasmas, rickettsias, chlamydias, and viruses. Pathogenic microbial antigens refer to substances that are derived from pathogens and have the function of triggering an immune response.

In order to more clearly describe the objects, features, and advantages of the present application, the present application will be described below in detail with reference to the drawings and specific embodiments, but the embodiments of the present application described below in detail are intended only to illustrate the content of the present application and do not constitute any limitation to the present application.

### Example 1. Construction of DNA Plasmid Template for UTR-Luciferase Reporter Gene Assay System

The T7 promoter sequence, the 5'-UTR sequences to be tested, the luciferase reporter gene sequence or the hEPO gene sequence, and the 3' UTR sequences were synthesized by GenScript and cloned to the xbaI/BamHI site of the puc57 plasmid vector.

The T7 promoter sequence was: TAATACGACTCACTATAGG (SEQ ID No. 19); the UTR sequences to be tested are shown in Table 1, and the sequence of the luciferase reporter gene was as follows:
atggaagacgccaaaaacataaagaaaggcccggcgccattctatccgctggaagatggaaccgctggagagcaactgcataaggctat gaagagatacgccctggttcctggaacaattgcttttacagatgcacatatcgaggtggacatcacttacgctgagtacttcgaaatgtccgtt cggttggcagaagctatgaaacgatatgggctgaatacaaatcacagaatcgtcgtatgcagtgaaaactctcttcaattctttatgccggtgtt gggcgcgttatttatcggagttgcagttgcgcccgcgaacgacatttataatgaacgtgaattgctcaacagtatgggcatttcgcagcctac cgtggtgttcgtttccaaaaaggggttgcaaaaaattttgaacgtgcaaaaaaagctcccaatcatccaaaaaattattatcatggattctaaaa cggattaccagggatttcagtcgatgtacacgttcgtcacatctcatctacctcccggttttaatgaatacgattttgtgccagagtccttcgata gggacaagacaattgcactgatcatgaactcctctggatctactggtctgcctaaaggtgtcgctctgcctcatagaactgcctgcgtgagatt ctcgcatgccagagatcctatttttggcaatcaaatcattccggatactgcgattttaagtgttgttccattccatcacggttttggaatgtttacta cactcggatatttgatatgtggatttcgagtcgtcttaatgtatagatttgaagaagagctgtttctgaggagccttcaggattacaagattcaaa gtgcgctgctggtgccaaccctattctccttcttcgccaaaagcactctgattgacaaatacgatttatctaatttacacgaaattgcttctggtgg cgctcccctctctaaggaagtcggggaagcggttgccaagaggttccatctgccaggtatcaggcaaggatatgggctcactgagactaca tcagctattctgattacacccgagggggatgataaaccgggcgcggtcggtaaagttgttccattttttgaagcgaaggttgtggatctggata ccgggaaaacgctgggcgttaatcaaagaggcgaactgtgtgtgagaggtcctatgattatgtccggttatgtaaacaatccggaagcgac caacgccttgattgacaaggatggatggctacattctggagacatagcttactgggacgaagacgaacacttcttcatcgttgaccgcctgaa gtctctgattaagtacaaaggctatcaggtggctcccgctgaattggaatccatcttgctccaacaccccaacatcttcgacgcaggtgtcgc aggtcttcccgacgatgacgccggtgaacttcccgccgccgttgttgttttggagcacggaaagacgatgacggaaaaagagatcgtggat tacgtcgccagtcaagtaacaaccgcgaaaaagttgcgcggaggagttgtgtttgtggacgaagtaccgaaaggtcttaccggaaaactcg acgcaagaaaaatcagagagatcctcataaaggccaagaagggcggaaagatcgccgtgtaa (SEQ ID No. 20); the hEPO gene sequence was NCBI Gene 2056; the 3'-UTR sequences are shown in Table 4.

The 5'-UTR sequences used in the present application are shown in Table 1:

**Table 1. Novel artificial 5'-untranslated region elements**

| Sequence No. | Name of novel artificial 5'-untranslated region element | Sequence (5'-3') |
|---|---|---|
| 1 | UTR1 (SEQ ID No.1) | AGGAATAACGAGACACCGTCTAGCACTTGCC CGTCAGTCCACAGTGCCCGAATCGCCCGCTG GTTTAAGTGCAGCCTGTCAATCAGAGCAGCT |
| | | CGAGGCCCGCCCAAGGCCGTGCTGCGTTTAC GTCACTTAGCCCGCGTAATCGATAAGGAAAC CCGGACGCTGCTGCCCAGGCGGCCGGGAAG GCCACC |
| 2 | UTR2 (SEQ ID No. 2) | AGGAATAACGAGACACCGTCTAGCACTTGCC CGTCAGTCCTACAGTGCCCGAAGCCACC |
| 3 | UTR3 (SEQ ID No. 3) | AGGAATAAAGGAAACCCGGACGCTGCTGCC CAGGCGGCCGGGAAGGCCACC |
| 4 | UTR4 (SEQ ID No. 4) | AGGAATAATCGCCCGCTGGTTTAAGTGCAGC CTGTCAATCAGAGCAGCTCTCGAGGCCCCGC CCAAGGCCGTGCTGCGTTTCTCCTTACGTCA CTTCCTCTCCAGCCCCTGCGTAATCGATAGCC ACC |
| 5 | UTR5 (SEQ ID No. 5) | AGGAATAATCGCCCGCTGGTTTAAGTGCAGC CTGTCAATCAGAGCAGCTCGAGGCCCGCCCA AGGCCGTGCTGCGTTTACGTCACTTAGCCCG CGTAATCGATAGCCACC |
| 6 | UTR6 (SEQ ID No. 6) | AGGAATAACGAGACACCGTCTAGCACTTCCC GCCCGTCAGTCCTTCACAGTGCCCGAAGCCA CC |
| 7 | UTR7 (SEQ ID No. 7) | AGGAATAAAGGAAACCCGGACGCTGCTGCC CCTTTCTTTTTTTCAGGCGGCCGGGAAGGCC ACC |
| 8 | UTR8 (SEQ ID No. 8) | AGGAATAACTGCCCGACAAAATACATCAGAA TTTAAGAACAATATCGGATCGGCCACC |
| 9 | UTR9 (SEQ ID No. 9) | AGGAATAAATTAAAAAATATATATATCGGATC AAATTGGGGGTACTTAATACCTGCGCCACC |
| 10 | UTR10 (SEQ ID No. 10) | AGGAATAAACCAACAACCAACAACAACATCC ACACCAACAACAACGCTGAAAGTGGTGTTTG CTTACCAGAAGGGCACACTTATCTAATTTGGG |
| | | GTATCGCGCCACC |
| 11 | UTR11 (SEQ ID No. 11) | AGGAATAAACCAACAACCAACAACAACATCC ACACCAACAACAACGCTGAAAGTGGTGGCC ACC |
| 12 | UTR12 (SEQ ID No. 12) | AGGAATAATTTGCTTACCAGAAGGGCACACT TATCTAATTTGGGGTATCGCGCCACC |
| 13 | UTR13 (SEQ ID No. 13) | AGGAATAACACACCCACTACACCTACACATAT CTACTCACCTACACACTAATATTTGCTTACCA GAAGGGCACACTTATCTAATTTGGGGTATCGC GCCACC |
| 14 | Ctrl (SEQ ID No. 14) | AGGAATAAACTAGTATTCTTCTGGTCCC CACAGACTCAGAGAGAACCCGCCACC |

### Example 2. Synthesis and Purification of UTR-Luciferase Reporter Gene mRNAs

Using the DNA plasmids (obtained in Example 1) with the candidate UTRs as templates, linear DNAs were amplified by PCR as templates for *in vitro* transcription of mRNAs. The conditions for the PCR reactions and the *in vitro* transcription of mRNAs are shown in Table 2 and Table 3, respectively.

**Table 2. The conditions for the PCR amplification reactions of the linear DNA templates**

| **Component** | **Volume (µL)** |
|---|---|
| DNA plasmid template (10 ng/µL) | 2 |
| 2 × Phanta Max Buffer | 50 |
| dNTP mix (10 mM each) | 2 |
| F primer (10 µM) | 2 |
| R primer (10 µM) | 2 |
| Phanta Max Super-Fidelity DNA Polymerase | 2 |
| H₂O (up to 100 µL) | 40 |

95 °C for 30 seconds; 95 °C for 15 seconds, 68 °C for 15 seconds, 72 °C for 90 seconds, 30 cycles; 72 °C for 5 minutes, 6 °C Hold. The PCR products were purified by centrifugation using a purification column.
Upstream primer: CGACGGCCAGAGAATTCGAGCTCGGTACCTCGCGAATACATC (SEQ ID No. 15);
Downstream primer: GCCGCCCACTCAGACTTTATTCAAAG (SEQ ID No. 16).

**Table 3. The conditions for the in vitro transcription (IVT) reaction of mRNAs**

| | |
|---|---|
| ATP (100 mM) | 3 µL |
| GTP (100 mM) | 3 µL |
| CTP (100 mM) | 3 µL |
| 5-OMe-UTP (100 mM) | 3 µL |
| 3-OMe-GAG | 2.4 µL |
| 10×Buffer | 4 µL |
| RNase inhibitor | 2 µL |
| T7 RNA polymerase | 2 µL |
| PPase | 0.8 µL |
| DNA template | 1 µg |
| Enzyme-free water | Added to 40 µL |
| 37 °C, 2 hours | |
| DNase | 1 µL |
| 37 °C, 15 minutes | |

The transcription products were purified as follows using NucleoSpin RNA Clean-up (740948.250, Macherey-Nagel) to give the mRNAs of interest:
1) 60 µL of enzyme-free water was added to 40 µL of IVT product to make a 100 µL sample.
2) 300 µL of Buffer RA1 was well mixed with 300 µL of absolute ethanol, and the above 100 µL sample was added. The mixture was well mixed by pipetting, added onto a purification column, and centrifuged at 8000 g for 30 s, and the effluent was discarded.
3) 700 µL of Buffer RA3 was added onto the purification column and centrifuged at 8000 g for 30 s, and the effluent was discarded.
4) 350 µL of Buffer RA3 was added onto the purification column and centrifuged at 8000 g for 2 min, and the effluent was discarded.
5) The purification column was placed above a 1.5 mL sterile enzyme-free centrifuge tube. 50 µL of enzyme-free water was added onto the purification column and centrifuged at 13,000 g for 1 min. The effluent was transferred to a new 1.5 mL sterile enzyme-free centrifuge tube, and a solution of the mRNA of interest was obtained.

### Example 3. mRNA Transfection and Luciferase Activity Assay

Adherently cultured HEK293 cells (ATCC) were digested, plated onto a 96-well plate, and transfected with the mRNAs to be tested using lipo3000. After 24 h of transfection, the cells were assayed for luciferase activity. The specific steps were as follows:
1) The adherently cultured HEK293 cells were washed with PBS 2 times, and 1 mL of 0.25% trypsin was added. After about 1 min of digestion in a 37 °C incubator, a medium (DMEM + 10% FBS) was added to stop the digestion.
2) The mixture was centrifuged at 300 g for 5 min, and the cells were collected and resuspended in a medium (DMEM + 10% FBS). Live cells were counted using trypan blue.
3) The cells were diluted to 3 × 10⁵ cells/mL with DMEM complete medium, plated onto a 96-well plate at 100 µL/well (about 30,000 cells/well), and cultured overnight in a 37 °C incubator.
4) An mRNA transfection dilution and a lipo3000 transfection dilution were mixed in a 1:1 ratio to form a lipo3000-mRNA complex. 10 µL of the lipo3000-mRNA complex was added to the 96-well cell culture plate, with each group in triplicate (100 ng of mRNA/well). The culture plate was placed back into the 37 °C, CO₂ incubator and incubated for another 24 h.
5) One-Lite^{™} assay reagent (100 µL, equal to the volume of the cells to be tested) equilibrated to room temperature was added. The plate was left to stand at room temperature for 1 min to fully lyse the cells and was shaken on a microplate reader for 3 min, and then the cell contents were assayed for luciferase activity.

The luciferase activity is shown in FIG. 1. Compared to the control UTR (Ctrl in FIG. 1, BNT162b2 5' UTR) and UTR1, UTR2 and UTR4 showed more than 2 times higher luciferase expression levels than the control group, and UTR8 and UTR9 showed more than 4 times and even nearly 8 times higher luciferase expression levels than the control group; UTR10, UTR11, and UTR12 showed 2-4 times higher luciferase expression levels than the control UTR; UTR13 showed 4-6 times higher luciferase expression levels than the control UTR.

### Example 4. Candidate UTR-Driven Plasma hEPO Expression

The UTR-hEPO-mRNAs to be tested were synthesized by *in vitro* transcription (IVT), purified by column chromatography, and then encapsulated in LNPs. Lipid components and an RNA were first dissolved in an oil phase and an aqueous phase, respectively, to form a volume ratio of 1:3. The lipid components, including ALC-0315 (Jenkem, C10201), DSPC (AVT, S01005), cholesterol (AVT, CHO-HP/O01001), and ALC-0159 (Jenkem, 3295), were dissolved in ethanol (in a molar ratio of 50:10:38.5:1.5), and the mRNA was diluted in 10 mM sodium citrate (J.T.Baker, 3647-01). The prepared solutions were mixed using a microfluidic mixing device at a flow rate of 12 mL/min to obtain LNPs. The LNP-encapsulated UTR-hEPO-mRNAs to be tested were injected into the tail veins of ICR mice. After 6 h, plasma was collected and assayed for plasma hEPO concentration by ELISA (Human Erythropoietin ELISA Kit, Elabscience, E-EL-H3640c).

The results are shown in FIG. 2A. UTR8 and UTR13 showed higher hEPO expression levels in mice than the control UTR (Ctrl in FIG. 2A, BNT162b2 5' UTR). To test the compatibility of UTR8 and UTR13 with different 3' UTR sequences, the inventors replaced the 3' UTR of the HBA1 gene used in FIG. 2A with the 3' UTR sequence of BNT162b2 and added "asterisks" to the names of the three sequences with BNT162b2 3' UTR to distinguish them. The three sequences were named Ctrl*, UTR8*, and UTR13*. As shown in FIG. 2B, the UTR8* and UTR13* reporter vectors showed higher hEPO expression levels in mice than the control UTR (Ctrl* in FIG. 2B, BNT162b2 5' UTR and 3' UTR). According to the data in FIG. 2, in the hEPO animal model, both UTR8 and UTR13 outperformed the control UTR as 5' UTR sequence elements when combined with two different 3' UTR sequences, respectively.

**Table 4. The 3' UTR sequences used in this experiment**

| | | |
|---|---|---|
| 1 | BNT162b2 3'UTR (SEQ ID No. 17) | CTCGAGCTGGTACTGCATGCACGCAATGCTAGCTG CCCCTTTCCCGTCCTGGGTACCCCGAGTCTCCCCCG ACCTCGGGTCCCAGGTATGCTCCCACCTCCACCTG CCCCACTCACCACCTCTGCTAGTTCCAGACACCTC CCAAGCACGCAGCAATGCAGCTCAAAACGCTTAGC CTAGCCACACCCCCACGGGAAACAGCAGTGATTAA CCTTTAGCAATAAACGAAAGTTTAACTAAGCTATAC TAACCCCAGGGTTGGTCAATTTCGTGCCAGCCACA CCCTGGAGCTAGCA |
| 2 | HBA1 3'UTR (SEQ ID No. 18) | GCTGGAGCCTCGGTGGCCATGCTTCTTGCCCCTTG GGCCTCCCCCCAGCCCCTCCTCCCCTTCCTGCACC |
| | | CGTACCCCCGTGGTCTTTGAATAAAGTCTGAGTGG GCGGC |

### Example 5. Candidate UTR-mRNA Solution Stability Tests

In this experiment, the stability of solutions of the UTR-mRNAs to be tested was tested by forced degradation. Specifically, each of the UTR-mRNAs to be tested was dissolved in a forced degradation solution (500 mM CHES, 100 mM MgCl2, pH 10), and samples of the UTR-mRNA solutions were taken at different time points (0.5 h, 1 h, 1.5 h, 2 h, 3 h, 5 h, 9 h, and 24 h); after degradation was neutralized (the components of the neutralization solution: 5 µL of 1 M Tris-HCl pH 7 + 1 µL of 500 mM EDTA-Na pH 8.0), mRNA integrity tests were performed using capillary electrophoresis (Agilent 5200).

The results are shown in FIG. 3. UTR8 and UTR13 have similar half-lives to the control UTR mRNA in solution. This indicates that the candidate UTRs did not affect the half-life of the mRNA and can improve the level of mRNA protein expression.

The present application is described above in detail so that those skilled in the art can understand the mechanisms and content of the present application and implement it. However, the description does not limit the protection scope of the present application, and any equivalent changes or modifications made in line with the spirit of the present application shall fall within the protection scope of the present application.

### Industrial Applicability

Compared to UTRs disclosed in the prior art, the 5'-untranslated region elements of the present application can make nucleic acid molecules comprising same have at least 25%, at least 50%, at least 75%, at least 100%, or at least 150% higher protein expression performance and at least 50%, at least 100%, at least 150%, at least 200%, at least 250%, at least 300%, at least 350%, at least 400%, at least 500%, at least 600%, at least 700%, or at least 800% higher expression efficiency. Therefore, the elements can be used for constructing artificial nucleic acid molecules with high expression performance and have excellent application prospects in the development of nucleic acid therapeutic drugs.

## Claims

1. A novel artificial 5'-untranslated region element, wherein the element is formed by operably linking the following motifs:
(1) a transcription-enhancing sequence, a Kozac sequence, and the full-length sequence of the 5'-untranslated region of a naturally highly expressed gene; or
(2) a transcription-enhancing sequence, a Kozac sequence, and a fragment sequence of the 5'-untranslated region of a naturally highly expressed gene, wherein the fragment sequence of the 5'-untranslated region does not comprise a TOP motif site or a uORF sequence; or
(3) a transcription-enhancing sequence, a Kozac sequence, a non-structural sequence, and a fragment sequence of the 5'-untranslated region of a naturally highly expressed gene, wherein the fragment sequence of the 5'-untranslated region does not comprise a TOP motif site or a uORF sequence.

2. The novel artificial 5'-untranslated region element according to claim 1, wherein the motifs are linked in the direction from 5' to 3' in the following order:
(1) the transcription-enhancing sequence, the full-length sequence of the 5'-untranslated region of the naturally highly expressed gene, and the Kozac sequence; or
(2) the transcription-enhancing sequence, the fragment sequence of the 5'-untranslated region of the naturally highly expressed gene free of the TOP motif site and the uORF sequence, and the Kozac sequence; or
(3) the transcription-enhancing sequence, the fragment sequence of the 5'-untranslated region of the naturally highly expressed gene free of the TOP motif site and the uORF sequence, the non-structural sequence, and the Kozac sequence.

3. The novel artificial 5'-untranslated region element according to claim 1 or 2, wherein the naturally highly expressed gene is selected from a naturally highly expressed gene of a mammal, bacterium, or virus.

4. The novel artificial 5'-untranslated region element according to claim 3, wherein the naturally highly expressed gene is selected from the ribosomal protein S25 gene, the actin β gene, the hemoglobin subunit α gene, the hemoglobin subunit β gene, the complement factor 3 gene, the cytochrome B-245 α gene, the human TM4SF1 gene, the human TXNIP gene, the human RPS11 gene, or the like, or a mutant thereof.

5. The novel artificial 5'-untranslated region element according to claim 4, wherein the mutant is cut from the 5' end, the 3' end, or any segment between the 5' end and the 3' end of the 5' UTR of the natural gene; the mutant is 50 nt to 200 nt (nucleotides) in length; the TOP motif therein is mutated into CTTT or CTT or CT or C; the uORF sequence of ATG therein is mutated into ATT or the three nucleotides ATG are completely deleted; no other sequence exists at the 5' end and/or the 3' end of the mutant or a non-structural nucleic acid sequence is linked to the 5' end and/or the 3' end of the mutant.

6. The novel artificial 5'-untranslated region element according to claim 1, wherein the fragment sequence of the 5'-untranslated region is cut from the 5' end, the 3' end, or any segment between the 5' end and the 3' end of the 5' UTR of the natural gene; the fragment is 50 nt to 200 nt (nucleotides) in length; in the fragment, the TOP motif site is mutated into CTTT or CTT or CT or C or the TOP motif sequence is completely deleted; in the fragment, the uORF sequence is mutated into ATT or the three nucleotides ATG are completely deleted; no other sequence exists at the 5' end and/or the 3' end of the fragment or a non-structural nucleic acid sequence is linked to the 5' end and/or the 3' end of the fragment.

7. The novel artificial 5'-untranslated region element according to claim 1, wherein the transcription-enhancing sequence is selected from AATAA, ATAAT, or AATAAA.

8. The novel artificial 5'-untranslated region element according to claim 1, wherein the Kozac sequence is GCCACC.

9. The novel artificial 5'-untranslated region element according to claim 1, wherein the non-structural sequence is 30 nt to 50 nt in length, comprises 80% or more AC and 20% or less T, and does not comprise G; the non-structural sequence is preferably CACACCCACTACACCTACACATATCTACTCACCTACACACTAATA (SEQ ID No. 21) or CATACCCAAATTTATATCTACATCTAAACCCAATTATTACCC (SEQ ID No. 22) or ACCACACACTTATCTACACAACTCAATCC (SEQ ID No. 23) or TCCACCCAACCCACTACTAATACCCACAACCCAACACCC (SEQ ID No. 24) or AACCCACACACTCACCTACTCATCCA (SEQ ID No. 25); the non-structural sequence is more preferably CACACCCACTACACCTACACATATCTACTCACCTACACACTAATA (SEQ ID No. 21).

10. The novel artificial 5'-untranslated region element according to claim 1, wherein the novel artificial 5'-untranslated region element is 50 nt or more in length and consists of only natural nucleotides.

11. An artificial nucleic acid molecule, wherein the artificial nucleic acid molecule comprises:
(1) at least one said novel artificial 5'-untranslated region element according to any of claims 1 to 10;
(2) at least one open reading frame; and
(3) at least one 3'-untranslated region element.

12. The artificial nucleic acid molecule according to claim 11, wherein the artificial nucleic acid molecule further comprises a nucleic acid sequence encoding a signal peptide.

13. The artificial nucleic acid molecule according to claim 11, wherein the artificial nucleic acid molecule further comprises a poly(A) sequence or a poly(A) signal sequence.

14. The artificial nucleic acid molecule according to any of claims 11 to 13, wherein the elements are linked directly or by linkers.

15. The artificial nucleic acid molecule according to any of claims 11 to 13, having at least 50%, at least 100%, at least 150%, at least 200%, at least 250%, at least 300%, at least 350%, at least 400%, at least 500%, at least 600%, at least 700%, or at least 800% higher expression efficiency than nucleic acid molecules that do not comprise the novel artificial 5'-untranslated region element according to claim 1.

16. A vector comprising the artificial nucleic acid molecule according to any of claims 11 to 15.

17. A host cell comprising the artificial nucleic acid molecule according to any of claims 11 to 15 or the vector according to claim 16.

18. A protein or polypeptide encoded by the artificial nucleic acid molecule according to any of claims 11 to 15 or the vector according to claim 16.

19. The protein or polypeptide according to claim 18, wherein the protein or polypeptide is a prophylactic protein or polypeptide or a therapeutic protein or polypeptide.

20. Use of the artificial nucleic acid molecule according to any of claims 11 to 15 or the vector according to claim 16 or the host cell according to claim 17 or the protein or polypeptide according to any of claims 18 to 19 in the preparation of a medicament.

21. A medicament comprising the artificial nucleic acid molecule according to any of claims 11 to 15, or the vector according to claim 16, or the host cell according to claim 17, or the protein or polypeptide according to any of claims 18 to 19.

22. The medicament according to claim 21, further comprising a pharmaceutically acceptable carrier or auxiliary material.
